# EUROPÄISCHE PATENTSCHRIFT

(11) **EP 1 415 978 B1**
(45) Veröffentlichungstag und Bekanntmachung des Hinweises auf die Patenterteilung: **22.02.2006**
(21) Anmeldenummer: 03019902.0
(22) Anmeldetag: 02.09.2003
(51) Int. Cl.: C07C 69/78, C08K 5/101

(54) **Benzoesäureisodecylestergemische, Herstellung und deren Verwendung**
Benzoic acid isodecyl ester mixtures, process for their preparation and their use
Mélanges d'esters isodécyliques d'acide benzoique, procédé de leur préparation et leur utilisation

(30) Priorität: 26.10.2002 DE 10249912
(43) Veröffentlichungstag der Anmeldung: 06.05.2004
(73) Patentinhaber: Oxeno Olefinchemie GmbH, 45772 Marl (DE)
(72) Erfinder: Koch, Jürgen, 45721 Haltern am See (DE); Grass, Michael, Dr., 45721 Haltern am See (DE)

(56) Entgegenhaltungen:
- WO-A-97/39060
- DE-A- 1 962 500
- US-A- 5 236 987

## Beschreibung

Die Erfindung betrifft Benzoesäureester des 2-Propylheptanols, 2-Propyl-4-methyl-hexanols, 2-Propyl-5-methyl-hexanols, 2-Isopropyl-4-methyl-hexanols und/oder 2-Isopropyl-5-methylhexanols, deren Gemische mit Phthalsäurealkylestern, Adipinsäurealkylestern oder Cyclohexandicarbonsäurealkylestern sowie die Verwendung dieser Gemische.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Düsononylphthalat (DINP) und Düsodecylphthalat (DIDP) Verwendung finden. Mit zunehmender Kettenlänge der Ester steigen die Löse- bzw. Geliertemperaturen und somit die Verarbeitungstemperaturen des Weich-PVC an. Die Verarbeitungstemperaturen können durch Zusatz von sogenannten Schnellgelierern wie beispielsweise die kurzkettigen Phthalate Dibutylphthalat (DBP), Diisobutylphthalat (DIBP), Benzylbutylphthalat (BBP) oder Diisoheptylphthalat (DIHP) wieder reduziert werden. Neben den kurzkettigen Phthalaten können auch Dibenzoesäureester wie Dipropylenglycoldibenzoate o.ä. zum gleichen Zwecke eingesetzt werden.

Diese Schnellgelierer zeigen oftmals die Eigenschaft, in PVC-Plastisolen auf Grund ihrer hohen Solvatationskraft zu einem starken Viskositätsanstieg mit der Zeit zu führen. Dies muss in vielen Fällen wieder durch Zugabe von viskositätsreduzierenden Agenzien kompensiert werden.

Bei der Herstellung von PVC-Plastisolen ist in der Regel eine niedrige Viskosität und eine möglichst niedrige Geliertemperatur gefordert. Darüber hinaus wird eine hohe Lagerstabilität, d. h. ein geringer Viskositätsanstieg des Plastisols mit der Zeit gewünscht.

Eine hohe Viskosität wäre bei der maschinellen Verarbeitung des Plastisols nachteilig; eine zu hohe Geliertemperatur würde zu reduzierter Produktionsgeschwindigkeit führen.

Weichmacher, die sowohl die Geliertemperatur in einer Formulierung signifikant absenken als auch die Viskosität des Plastisols auch nach mehrtägiger Lagerdauer auf einem niedrigen Niveau halten, sind bisher kaum bekannt. Kürzlich wurde 2-Ethylhexylbenzoat als ein Produkt, welches diese Anforderungen erfüllen könnte, vorgestellt [Bohnert, Stanhope, J. Vinyl Addit. Technol. (2000), 6(3), 146-149]. Diese Verbindung hat allerdings einen vergleichsweise hohen Dampfdruck, was oft zu nicht akzeptablen Verlusten während der Verarbeitung führt.

In DE 19 62 500 wird die Verwendung eines Gemisches von längerkettigen Estern der Benzoe- und Phthalsäure zur Herstellung von Plastisolen offenbart. Zur Herstellung der Benzoesäureester wird bevorzugt 3,5,5-Trimethylhexanol eingesetzt; über die einzusetzenden Phthalsäurediester werden keine genauen Angaben gemacht.

In US 5 236 987 wird die Verwendung von Benzoesäureestern von Isodecanolen, die ausschließlich Methylverzweigungen aufweisen, als Weichmacher bzw. Gelierungsmittel für PVC offenbart.

Die Verwendung von Benzoesäurealkylestern deren Alkylgruppe 11-14 C-Atome aufweisen, ist aus WO 97/39060 bekannt. Längerkettige Ester besitzen zwar eine niedrige Flüchtigkeit, jedoch wieder schlechtere Geliereigenschaften .

Die vorgenannten Weichmachersysteme weisen in PVC neben der Flüchtigkeit in ihren Geliereigenschaften, in der Kälteflexibilität und der Lagerstabilität noch Verbesserungspotential auf.

Es bestand daher die Aufgabe, neue Weichmacher für Kunststoffe wie z. B. für PVC zu finden, die eine preiswerte Rohstoffbasis haben und gleichwertige oder verbesserte Weichmachereigenschaften wie beispielsweise ein verbessertes Kälteflexibilisierungsvermögen bei niedrigem Viskositätsniveau der entsprechenden Plastisole aufweisen.

Bei der Herstellung von Weich-PVC ist auf eine gute Gelierfähigkeit und eine niedrige Flüchtigkeit des Weichmachers zu achten. Dies wird durch die Wechselwirkungen zwischen Weichmacher und PVC-Polymerkette beeinflusst, so dass auch geringe strukturelle Änderungen der molekularen Struktur des Weichmachermoleküls anwendungstechnisch große Eigenschaftsänderungen bewirken können.

Überraschender Weise wurde gefunden, das Benzoesäureester des 2-Propylheptanols, 2-Propyl-4-methyl-hexanols, 2-Propyl-5-methyl-hexanols, 2-Isopropyl-4-methyl-hexanols und/oder 2-Isopropyl-5-methyl-hexanols allein oder im Gemisch mit Phthalsäureestern und/oder Adipinsäuredialkylestern und/oder Cyclohexyldicarbonsäureestem die gewünschten anwendungstechnischen Profile aufweisen. Hierbei wurde fest gestellt, dass die anwendungstechnischen Eigenschaften des Estergemisches mit zunehmendem Gehalt an Benzoesäure-2-propylheptylester sukzessive verbessert werden können. Die anwendungstechnischen Eigenschaften sind vermutlich auf die im Vergleich zu US 5 236 987 (i.d. R. mehrere Methylverzweigungen pro Alkylgruppe), geringere Anzahl von Verzweigungen in den Estergruppen zurückzuführen.

Gegenstand der vorliegenden Erfindung sind daher Gemische isomerer Benzoesäuredecylester, enthaltend
50 - 99 %, bevorzugt 70 - 99 %, besonders bevorzugt 85 - 99 % Benzoesäure-2-propylheptylester und
1 - 50 %, bevorzugt 1 - 30 %, besonders bevorzugt 1 - 15 % Benzoesäure-2-isopropyl-4-methyl-hexylester und/oder Benzoesäure-2-isopropyl-5-methyl-hexylester, Benzoesäure-2-propyl-4-methylhexylester, Benzoesäure-2-propyl-5-methylhexylester.

Die erfindungsgemäßen Benzoate können technisch einfach durch Aldolkondensation der C5-Aldehyde n-Valeraldehyd (= n-Pentanal), iso-Valeraldehyd (2-Methylbutanal) und 3-Methylbutanal, mit anschließender Wasserabspaltung, Hydrierung und Veresterung mit Benzoesäure erhalten werden.

N- bzw. iso-Valeraldehyd können wiederum beispielsweise durch Hydroformylierung von 1-Buten hergestellt werden. Bei dieser Reaktion fällt n- und iso-Valeraldehyd in wechselnden Verhältnissen an. Wird ein solches Gemisch einer Aldolkondensation unterworfen, so werden verschiedene substituierte Produkte erhalten; die anschließende Veresterung mit Benzoesäure ändert an diesen Isomerenverhältnissen nichts. Die Hydroformylierung von Isobuten macht 3-Methylbutanal zugänglich.

Die Synthese der Isodecanole kann durch folgende Schritte erfolgen:
a) ein C₄-Olefin oder ein C₄-Olefingemisch wird zu den entsprechenden C₅-Aldehyden hydroformyliert
b) die unter a) entstandenen Aldehyde werden zu Decenalen aldolkondensiert
c) die im Schritt b) entstandenen Decenale werden zu Decanolen hydriert

Für die Herstellung der Decanolgemische werden 1-Buten, 2-Butene, Isobuten oder Gemische dieser Olefine als Ausgangsstoffe verwendet. Die Hydroformylierung dieser Gemische kann nach verschiedenen Verfahren durchgeführt werden.

Die Synthese der Isodecylalkohole aus einem C₄-Olefin oder einem C₄-Olefingemisch ist in der Regel wirtschaftlicher als der konventionelle Weg über die Trimerisierung von Propylen mit anschließender Hydroformylierung und Hydrierung, bei dem überwiegend methylverzweigte Isodecanolgemische entstehen.

In der Regel werden für die Hydroformylierung Kobalt- oder Rhodiumkatalysatoren, unmodifiziert oder modifiziert eingesetzt.

Die Hydroformylierung von Isobuten zu 3-Methylbutanal wird beispielsweise in folgender Literaturstelle beschrieben (V. Y. Gankin, L. S. Genender, D. M. Rudkovskii, USSR Zh. Prikl. Khim. (Leningrad) (1968), 41 (10), S. 2275-81).

Die Hydroformylierung von linearen Butenen oder deren Gemischen wird beispielsweise in den Druckschriften EP 0 094 456, DE 196 17 178, EP 0 562 451oder EP 0 646 563 offenbart.

Die Aldolkondensation von n-Valeraldehyd, iso-Valeraldehyd, 3-Methylbutanal oder eines Gemisches von C₅-Aldehyden erfolgt auf üblicher Weise unter Einwirkung basischer Katalysatoren. Als Katalysatoren finden Alkalicarbonate oder Alkalihydroxide, insbesondere Verbindungen des Natriums oder Kaliums, oder Amine, vorzugsweise tertiäre Amine wie Triethylamin, Tri-n-propylamin, Tri-n-butylamin Anwendung. Man arbeitet bei Temperaturen von 60 bis 160 °C , insbesondere 80 bis 130 °C und bei Normaldruck oder bei bis etwa 1 MPa erhöhtem Druck. Die Reaktionszeit beträgt wenige Minuten bis zu mehreren Stunden und ist insbesondere abhängig vom Katalysatortyp und Reaktionstemperatur.

Die Aldolkondensation von C₅-Aldehyden in Rührreaktoren wird beispielsweise in WO 93/20034 beschrieben. Die Durchführung von Aldolkondensationen von Aldhyden in Rohrreaktoren wird beispielsweise in DE 199 57 522 offenbart.

Die durch Aldolkondensation der C₅-Aldehyde gewonnenen Decenale werden in reiner Form oder als Gemisch hydriert. Die Hydrierung wird bevorzugt in der Flüssigphase durchgeführt.

Zur Hydrierung können Katalysatoren oder Katalysatorsysteme verwendet werden, die sowohl olefinische Doppelbindungen als auch Carbonylgruppen hydrieren. Für die Hydrierung der α,β-ungesättigten Aldehyde sind insbesondere diejenigen Katalysatoren geeignet, die in der Technik für die Hydrierung von 2-Ethylhex-2-enal zu 2-Ethylhexanol eingesetzt werden.

Man kann zur Hydrierung z. B. Kupfer/Nickel-, Kupfer/Chrom-, Kupfer/Chrom/Nickel-, Zink/Chrom-, Nickel/Molybdän-Katalysatoren verwenden. Es können auch Kombinationen von zwei oder mehreren Katalysatoren verwendet werden. Die Katalysatoren können trägerfrei sein, oder die hydrieraktiven Stoffe bzw. ihre Vorläufer können auf Träger, wie beispielsweise Siliciumdioxid oder Aluminiumdioxid, aufgebracht sein.

Bevorzugte Katalysatoren, an denen die α,β-ungesättigten Aldehyde hydriert werden, enthalten jeweils 0,3- 15 Massen-% Kupfer und Nickel sowie als Aktivatoren 0,05 - 3,5 Massen-% Chrom und vorteilhaft 0,01 - 1,6 Massen-%, vorzugsweise 0,02 - 1,2 Massen-% einer Alkalikomponente auf einem Trägermaterial, vorzugsweise Aluminiumoxid und Siliciumdioxid. Die Mengenangaben beziehen sich auf den noch nicht reduzierten Katalysator. Die Alkalikomponente ist optional.

Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudate oder Ringen. Sie werden zweckmäßig vor ihrem Einsatz aktiviert, z. B. durch Erhitzen im Wasserstoffstrom.

Die Hydrierung, bevorzugt eine Flüssigphasenhydrierung, wird im Allgemeinen unter einem Gesamtdruck von 5 bis 200 bar, insbesondere von 5 bis 30 bar ganz besonders 15 bis 25 bar durchgeführt. Eine Hydrierung in der Gasphase kann auch bei niedrigeren Drücken durchgeführt werden, mit entsprechend großen Gasvolumina. Werden mehrere Hydrierungsreaktoren eingesetzt, können die Gesamtdrücke in den einzelnen Reaktoren innerhalb der genannten Druckgrenzen gleich oder verschieden sein.

Die Reaktionstemperaturen liegen bei Hydrierung in flüssiger oder gasförmiger Phase in der Regel zwischen 120 und 220 °C, insbesondere zwischen 140 und 180 °C.
Beispiele für solche Hydrierungen sind in den Patentanmeldungen EP 0 470 344 und EP 0 326 674 beschrieben.

Optional kann die Hydrierung von Decenalen zu Decanolen zweistufig durchgeführt werden. Dabei wird in der ersten Stufe beispielsweise an einem Palladiumkatalysator die olefinische Doppelbindung und in der zweiten Stufe an einem der oben genannten Kontakte die Carbonylgruppe hydriert.

Ausgehend von C₄-Olefinen entstehen Decanolgemische, die im Wesentlichen einen oder mehreren der folgenden Stoffe enthalten:

2-Propylheptanol, 4-Methyl-2-propyl-hexanol, 5-Methyl-2-propyl-hexanol, 2-isopropyl-4-methyl-hexanol, 2-isopropyl-5-methyl-hexanol. Die aufgezählten Decanole bestehen jeweils aus mindestens zwei Stereoisomeren. Gemische dieser Komponenten werden im Folgenden als Isodecylalkohol oder Isodecanol bezeichnet.

Die Zusammensetzung der Decanolgemische ist, wie bereits erwähnt, abhängig vom Einsatzstoff und dem Hydroformylierungsverfahren. Alle Decanolgemische, die auf beschriebener Weise aus C₄-Olefinen gewonnen werden, können zur Herstellung der erfindungsgemäßen Ester eingesetzt werden. Bevorzugte Decanolgemische sind diejenigen, die zu 50 - 99 %, insbesondere zu 70 - 99 %, besonders bevorzugt zu 85 - 99 Gew.%, insbesondere 95 - 99 % aus 2-Propylheptanol bestehen. Je höher der Anteil an 2-Propylheptanol in der Mischung ist, desto vorteilhaftere Eigenschaften stellen sich im hieraus hergestellten Benzoesäureester ein.

Die erfindungsgemäßen Benzoatgemische können als Viskositätserniedriger und schnellgelierende Weichmacher verwendet werden und zeichnen sich gegenüber bekannten Systemen bei der Modifizierung von Kunststoffen wie PVC durch eine sehr vorteilhafte Kombination aus geringer Flüchtigkeit, guter Gelierfähigkeit, guter Kälteflexibilisierung und geringem Viskositätsanstieg in Plastisolen aus.

Die erfindungsgemäßen Benzoesäureester können durch Veresterung von Benzoesäure mit den entsprechenden Alkoholen oder durch Umestem von anderen, niedermolekularen Benzoesäureestern hergestellt werden.

Beide Varianten sind dem Fachmann gut bekannt (z. B. "Organikum" , Wiley-VCH, 21. Auflage). Zur Umesterung können z. B. ein oder mehrere Benzoesäurealkylester, bevorzugt Benzoesäuremethylester, Benzoesäureethylester, Benzoesäurepropylester, Benzoesäureisobutylester, Benzoesäureamylester und/oder Benzoesäurebutylester eingesetzt werden.

Weiterhin sind Gegenstände der vorliegenden Erfindung Gemische aus den erfindungsgemäßen Benzoesäureisodecylestern mit jeweils Phthalsäuredialkylestern, bevorzugt Phthalsäuredüsononylester, oder mit Adipinsäuredialkylestern, bevorzugt den Adipinsäuredüsononylestern oder mit Cyclohexandicarbonsäwealkylestern, bevorzugt den Cyclohexandicarbonsäurediisononylester, wobei die Dialkylester 4-13, bevorzugt 8 bis 10 Kohlenstoffatome in der vom eingesetzten Alkohol stammenden Alkylgruppe, enthalten.

Diese Gemische enthalten 5 - 90, bevorzugt 5 - 50, besonders bevorzugt 10 - 40 Gew.-% der erfindungsgemäßen Benzoesäweisodecylestergemische und 10 - 95, bevorzugt 50 - 95, besonders bevorzugt 60 - 90 Gew.-% der Phthalsäuredialkylester, Adipinsäuredialkylester, Cyclohexandicarbonsäurealkylester.

Die Gew.-%-Angaben der Benzoesäureisodecylestergemische und der weiteren Ester addieren sich auf 100 %. Die Gew.-%-Angaben der Benzoesäureisodecylesterisomere innerhalb des Benzoesäureisodecylestergemisches entsprechen den genannten Werten.

Bevorzugt werden zur Herstellung der erfindungsgemäß verwendeten Nonylphthalate und/oder -adipate und/oder Cyclohexyldicarbonsäureester, technische Nonanolgemische, d. h. Gemische der isomeren Alkohole, im folgenden Text als Isononanol oder Isononanolgemisch bezeichnet, eingesetzt.

Die Isomerenverteilung dieser Gemische wird durch die Art der Herstellung des verwendeten Nonylalkohols (Isononanol) bestimmt.

Isononanol wird durch Hydroformylierung von Octenen, die wiederum auf unterschiedliche Art erzeugt werden, hergestellt. Als Rohstoff hierzu dienen im allgemeinen technische C₄₋Ströme, die zunächst alle isomeren C₄-Olefine neben den gesättigten Butanen und ggf. Verunreinigungen wie C₃- und C₅-Olefinen und acetylenischen Verbindungen enthalten. Durch Oligomerisierung dieses Olefingemisches erhält man vorwiegend isomere Octengemische neben höheren Oligomeren wie C₁₂- und C₁₆-Olefingemischen.

Diese Octengemische werden zu den entsprechenden Aldehyden hydroformyliert und anschließend zum Alkohol hydriert.

Die Zusammensetzung, d. h. die Isomerenverteilung der technischen Nonanolgemische ist abhängig vom Ausgangsmaterial und von den Oligomerisierungs- und Hydroformylierungsverfahren. Zur Herstellung der erfindungsgemäßen Ester können alle diese Gemische eingesetzt werden. Bevorzugte Nonanolgemische sind diejenigen, die durch Hydroformylierung von C₈-Olefingemischen, erhalten durch Oligomerisierung von im Wesentlichem linearen Butenen an Nickelträgerkatalysatoren (z. B. OCTOL-Prozeß), in Gegenwart von unmodifizierten Kobaltverbindungen und anschließender Hydrierung des entkatalysierten Hydroformylierungsgemisches gewonnen wurden. Dabei beträgt der Anteil von Isobuten im Ausgangsstoff, bezogen auf den Gesamtbutengehalt, weniger als 5 Gew-%, vorzugsweise weniger als 3 Gew-%, besonders bevorzugt weniger als 1 Gew-%. Hierdurch wird erreicht, dass der Anteil stärker verzweigter Nonanol-Isomerer, u.a. auch der des 3,5,5-Trimethylhexanols, welches sich als wenig vorteilhaft gezeigt hat, deutlich zurück gedrängt wird. Erfindungsgemäße Gemische enthalten daher unter 10, bevorzugt unter 5, besonders bevorzugt unter 3, insbesondere unter 1 Gew-% Ester des 3,5,5-Trimethylhexanols. Diese Angaben beziehen sich auf die Alkoholgemische, die sich aus der Verseifung der erfindungsgemäßen Estergemische ergeben würden.

Es handelt sich hier um Alkoholmischungen, die neben den genannten Isononylalkoholen auch Alkohole mit 7 bis 15 Kohlenstoffatome (gemäß CAS-Definition) enthalten.

Erfindungsgemäße Gemische werden durch die Zusammensetzung der genannten Ester definiert, nicht durch Art oder Reihenfolge der Herstellung der Gemische. Gemische im Sinne der vorliegenden Erfindung liegen auch vor, wenn die genannten Ester im genannten Verhältnis gleichzeitig oder nacheinander mit einem weiteren Stoff wie Kunststoffen (z. B. PVC) gemischt werden.

Gegenstand der vorliegenden Erfindung ist auch ein Verfahren zur Herstellung eines Gemisches isomerer Benzoesäuredecyclester, enthaltend
50 - 99 % Benzoesäure-2-propylheptylester und
1 - 50% Benzoesäure-2-isopropyl-4-methyl-hexylester und/oder Benzoesäure-2-isopropyl-5-methyl-hexylester, Benzoesäure-2-propyl-4-methylhexylester, Benzoesäure-2-propyl-5-methylhexylester, dadurch gekennzeichnet, dass 2-Propylheptanol, 2-Isopropyl-4-methylhexanol und/oder 2-Isopropyl-5-methyl-hexanol, 2-Propyl-4-methylhexanol, 2-Propyl-5-methylhexanol mit Benzoesäure verestert werden.

Die Veresterung der Benzoesäure, Phthalsäure bzw. Phthalsäureanhydrid und/oder Adipinsäure und/oder Cyclohexandicarbonsäure bzw. deren Anhydriden mit einem Isodecanol bzw. einem der gewünschten Alkanole, insbesondere einem isomerenreinen Nonanol oder einem Isononanolgemisch zu den entsprechenden Estern kann autokatalytisch oder katalytisch, beispielsweise mit Brönstedt- oder Lewissäuren durchgeführt werden. Ganz gleich welche Art der Katalyse gewählt wird, es entsteht immer ein temperaturabhängiges Gleichgewicht zwischen den Einsatzstoffen (Säure und Alkohol) und den Produkten (Ester und Wasser). Um das Gleichgewicht zu Gunsten des Esters zu verschieben, kann ein Schleppmittel eingesetzt werden, mit dessen Hilfe das Reaktionswasser aus dem Ansatz entfernt wird. Da die zur Veresterung eingesetzten Alkoholgemische niedriger als die Benzoesäure und deren Ester sieden und mit Wasser eine Mischungslücke aufweisen, werden sie häufig als Schleppmittel eingesetzt, das nach Wasserabtrennung wieder in den Prozess zurückgeführt werden kann.

Der zur Bildung des Esters eingesetzte Alkohol bzw. das isomere Alkoholgemisch, das gleichzeitig als Schleppmittel dient, wird im Überschuss, bevorzugt 5 bis 50 %, insbesondere 10 bis 30 % der zur Bildung des Esters notwendigen Menge eingesetzt.

Als Veresterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität oft erst bei Temperaturen oberhalb 180°C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Die Katalysatorkonzentration hängt von der Art des Katalysators ab. Bei den bevorzugt eingesetzten Titanverbindungen beträgt diese 0,005 bis 1,0 Massen-% bezogen auf das Reaktionsgemisch, insbesondere 0,01 bis 0,5 Massen-%, ganz besonders 0,01 bis 0,1 Massen-%.

Die Reaktionstemperaturen liegen bei Verwendung von Titankatalysatoren zwischen 160 °C und 270 °C, vorzugsweise bei 180 bis 250 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers günstig, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei verminderten Druck durchgeführt. Beispielsweise wird bei der Umsetzung von Benzoesäure mit einem Gemisch isomerer Nonanole in einem Temperaturbereich von 170 °C bis 250 °C im Druckbereich von 1 bar bis 10 mbar gearbeitet.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen.

Die Rohestergemische, die neben dem/den Ester(n), Alkohol, Katalysator oder dessen Folgeprodukten und gegebenenfalls Nebenprodukte enthalten, werden nach an sich bekannten Verfahren aufgearbeitet. Die Aufarbeitung umfasst dabei folgende Schritte: Abtrennung des überschüssigen Alkohols und ggf. Leichtsieder, Neutralisation der vorhandenen Säuren, optional eine Wasserdampfdestillation, Umwandlung des Katalysators in einen leichtfiltrierbaren Rückstand, Abtrennung der Feststoffe und gegebenenfalls eine Trocknung. Dabei können je nach angewendetem Aufarbeitungsverfahren die Reihenfolge dieser Schritte verschieden sein.

Optional kann der gewünschte Ester, Nonylester oder das Gemisch der Ester aus dem Reaktionsgemisch, gegebenenfalls nach Neutralisation des Ansatzes, destillativ abgetrennt werden.

Alternativ können die erfindungsgemäßen Benzoate durch Umesterung eines Benzoesäureesters mit dem erforderlichen Decanol-Isomerengemisch gewonnen werden. Als Edukte werden Benzoesäureester eingesetzt, deren am O-Atom der Estergruppe gebundenen Alkylreste 1 - 9 C-Atome aufweisen. Diese Reste können aliphatisch, geradkettig oder verweigt, alicyclisch oder aromatisch sein. Eine oder mehrere Methylengruppen dieser AlkylReste können durch Sauerstoff substituiert sein. Es ist zweckmäßig, dass die dem Eduktester zugrunde liegenden Alkohole niedriger sieden als das eingesetzte Decanol oder Isodecanolgemisch. Ein bevorzugter Einsatzstoff ist Benzoesäuremethylester.

Die erfindungsgemäßen Estergemische können daher auch durch Umestern von Benzoesäuremethylester, Benzoesäureethylester, Benzoesäurepropanester, Benzoesäurebutanester mit 2-Propylheptanol, 2-Isopropyl-4-methyl-hexanol und/oder 2-Isopropyl-5-methyl-hexanol, 2-Propyl-4-methylhexanol, 2-Propyl-5-methylhexanol hergestellt werden.

Die Umesterung wird katalytisch, beispielsweise mit Brönstedt- oder Lewissäuren oder Basen, durchgeführt. Ganz gleich welcher Katalysator eingesetzt wird, es entsteht immer ein temperaturabhängiges Gleichgewicht zwischen den Einsatzstoffen (Alkylbenzoat und Isodecanolgemisch) und den Produkten (Isodecylestergemisch und freigesetzter Alkohol). Um das Gleichgewicht zu Gunsten des Isodecylestergemisches zu verschieben, wird der aus dem Eduktester entstehende Alkohol aus dem Reaktionsgemisch abdestilliert.

Es ist auch hier zweckmäßig, den Alkohol im Überschuss einzusetzen.
Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Weiterhin können basische Katalysatoren, wie beispielsweise Oxide, Hydroxide, Hydrogencarbonate, Carbonate oder Alkoholate von Alkali- oder Erdalkalimetallen verwendet werden. Aus dieser Gruppe werden bevorzugt Alkoholate, wie beispielsweise Natriummethylat eingesetzt. Alkoholate können auch in situ aus einem Alkalimetall und einem Decanol bzw. einem Isodecanolgemisch hergestellt werden.

Die Katalysatorkonzentration hängt von der Art des Katalysators ab. Sie liegt üblicherweise zwischen 0,005 bis 1,0 Massen-% bezogen auf das Reaktionsgemisch.

Die Reaktionstemperaturen für die Umesterung liegen üblicherweise zwischen 100 und 220 °C. Sie müssen mindestens so hoch sein, dass der aus dem Eduktester entstehende Alkohol bei dem vorgegebenen Druck, meistens Normaldruck, aus dem Reaktionsgemisch abdestillieren kann.

Die Umesterungsgemische können genauso wie für die Veresterungsgemische beschrieben aufgearbeitet werden.

Die erfindungsgemäßen Gemische können alleine oder in Kombination mit anderen Weichmachern in Kunststoffen eingearbeitet werden. Bevorzugte Kunststoffe sind PVC, PVB, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

Als Vertreter der obigen Gruppen seien beispielsweise folgende Kunststoffe genannt:
Polyacrylate mit gleichen oder verschiedenen Alkylresten mit 4 bis 10 C-Atomen, gebunden am Sauerstoffatom der Estergruppe, insbesondere mit dem n-Butyl-, n-Hexyl-, n-Octyl-, Isononyl- und 2-Ethylhexylrest, Polymethacrylat, Polymethylmethacrylat, Methylacrylat-Butylacrylat-Copolymere, Methylmethacrylat-Butylmethacrylat-Copolymere, EthylenVinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose.

Als PVC-Typen kommen Suspensions-, Masse-, Mikrosuspensions- oder bevorzugt Emulsions-PVC oder auch deren Mischungen in Frage. Neben den beschriebenen Estern der Cyclohexandicarbonsäure, Phthalsäure, Adipinsäure und Benzoesäure sowie weiteren Weichmachern können der Rezeptur zahlreiche weitere, dem Fachmann bekannte Komponenten beigefügt werden. Beispiele hierfür sind Füllstoffe, Pigmente, Stabilisatoren, Gleitmittel, Treibmittel, Kicker, Antioxidanzien, Biozide etc.

Bevorzugt werden die erfindungsgemäßen Gemische zur Herstellung von Plastisolen, insbesondere von denen des PVC, mit besonders vorteilhaften verarbeitungstechnischen Eigenschaften eingesetzt. Diese Plastisole können in zahlreichen Produkten wie beispielweise Kunstleder, Fußböden, Tapeten etc eingesetzt werden. Unter diesen Anwendungen besonders bevorzugt ist die Verwendung in cushion vinyl (CV)-Fußböden, hier insbesondere in der Deckschicht, wo eine weitere Verbesserung in der Fleckbeständigkeit ("Stain Resistance") bewirkt wird. Durch Verwendung der erfindungsgemäßen Gemische als Rezepturbestandteil können Plastisole mit niedriger Viskosität sowie erhöhter Lagerstabilität und gleichzeitig mit sehr guter Gelierung und weiter verbesserter Kälteflexibilisierung erhalten werden.

Weiterhin können die erfindungsgemäßen Benzoate oder die oben erwähnten erfindungsgemäßen Gemische mit Phthalaten, Adipaten und/oder Cyclohexandicarboxylaten als Flexibilisierungsmittel in Lacken, Farben, Tinten oder Klebstoffen bzw. Klebstoffkomponenten sowie Dichtungsmassen eingesetzt werden.
Die folgenden Beispiele sollen die Erfindung erläutern, ohne die Anwendungsbreite einzuschränken, die sich aus der Beschreibung und den Patentansprüchen ergibt.

### Beispiel 1: Herstellung eines Isodecyl-Alkoholgemisches als Ausgangsstoff für die erfindungsgemäßen Benzoesäureester

2-Propylheptanol und 2-Propyl-4-methyl-hexanol werden durch eine Literatur bekannte Synthesesequenz (Hydroformylierung von 1-Buten, nachfolgende Aldolisierung der erhaltenen Aldehyde und Hydrierung der Decenale) hergestellt. Durch Zugabe von reinem 2-Propylheptanol wird ein Verhältnis von 90 Gew.-% 2-Propylheptanol und 10 Gew.-% 2-Propyl-4-methyl-hexanol eingestellt.

### Beispiel 2: Herstellung des erfindungsgemäßen Benzoates

In einem 4-Liter-Destillationskolben mit aufgesetztem Wasserabscheider und Rückflusskühler sowie einem Probenahmestutzen und Thermometer wurden 976 g Benzoesäure (8 Mol), 1872 g Isodecyl-Alkoholgemisch aus Beispiel 1 (12 Mol) und 0,59 g Butyltitanat (0,06% bezogen auf die Säuremenge) eingewogen und unter Stickstoffatmosphäre zum Sieden erhitzt. Das bei der Veresterung anfallende Reaktionswasser wurde regelmäßig abgenommen. Als die Säurezahl nach etwa 3 Stunden unter 0,1 mg KOH/g sank, wurde der überschüssige Alkohol im Vakuum, über eine 10 cm Kolonne mit Raschigringen, abdestilliert. Danach wurde der Ansatz auf 80 °C abgekühlt und in einen 4 Liter Reaktionskolben mit Tauchrohr, aufgesetztem Tropftrichter und Kolonne umgefüllt, und an einer Claisenbrücke befestigt. Danach wurde mit 5 %iger Natronlauge neutralisiert (etwa 10facher Laugenüberschuss). Anschließend wurde unter Vakuum (10 mbar) auf 190 °C aufgeheizt. Dann wurden über den Tropftricher 8 % VE-Wasser, bezogen auf die eingesetzte Menge Rohester, bei konstanter Temperatur zugetropft. Nach Zugabe des Wassers wurde die Heizung abgestellt und unter Vakuum abgekühlt.

Der Ester wurde bei Raumtemperatur über eine Nutsche mit Filterpapier und Filterhilfsmittel filtriert. Gaschromatographisch konnte eine Reinheit des Esters von 99,9 % ermittelt werden.

### Beispiel 3: Herstellung von Isodecylbenzoat aus überwiegend methyl-verzweigtem Isodecanol (Vergleichsbeispiel)

Ein typischer Vertreter für eine praktisch ausschließlich mit Methylgruppen verzweigte C10-Alkoholmischung ist das Exxal 10, ein Isodecylalkoholgemisch der Firma ExxonMobil Chemical.
Die unter Beispiel 2 beschriebene Veresterung wird unter Verwendung von Exxal 10 an Stelle des dort verwendeten Isodecylalkohol-Gemisches wiederholt. Der entsprechende Ester hat eine Reinheit von 99,8 %.

### Beispiel 4: Herstellung von Plastisolen auf Basis der Benzoate aus Beispiel 2 und 3

In den Rezepturen A und B sind jeweils lediglich die beiden schnell gelierenden Weichmacher vertreten, um die Unterschiede zwischen diesen Typen stärker heraus zu arbeiten. Die Rezepturen C und D enthalten praxisrelevante Mischungen aus VESTINOL 9 (DINP der Fa. OXENO Olefinchemie GmbH) und Schnellgelierer in typischen Deckstrich-Formulierungen.
Die Einwaage der Komponenten ist der nachfolgenden Tabelle zu entnehmen.

**Tabelle 1: Rezepturen (Alle Angaben in phr (= Gewichtsteile pro 100 Teile PVC))**

| | **A** | **B** | **C** | **D** |
|---|---|---|---|---|
| VESTOLIT B 7021 (Emulsions-PVC) | 100 | 100 | 100 | 100 |
| VESTINOL 9 (DINP, *OXENO)* | 0 | 0 | 35 | 35 |
| Isodecylbenzoat (aus Beispiel 2, erfindungsgemäß) | 50 | 0 | 15 | 0 |
| Methylverzweigtes Isodecylbenzoat (aus Beispiel 3; Vergleichsbeispiel) | 0 | 50 | 0 | 15 |
| Drapex 39 (Co-Stabilisator, *Crompton)* | 3 | 3 | 3 | 3 |
| Mark CZ 140 (Ca/Zn-Stab., *Crompton)* | 1,5 | 1,5 | 1,5 | 1,5 |

Die Weichmacher wurden vor der Zugabe auf 25 °C temperiert. Die flüssigen Bestandteile wurden zuerst, dann die pulverförmigen in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

### Beispiel 5: Messung der Viskositäten der Plastisole

Die Messung der Viskositäten der in Beispiel 4 hergestellten Plastisole wurden in Anlehnung an die DIN 53 019 mit dem Rheometer Physica DSR 4000, welches über die Software US 200 gesteuert wird, wie folgt durchgeführt.
Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o.g. Software. Folgende Punkte wurden angesteuert:
- Eine Vorscherung von 100 s⁻¹ für den Zeitraum von 60 s, bei der keine Messwerte aufgenommen wurden
- Eine Abwärtsrampe, beginnend bei 200 s⁻¹ bis herunter zu 0,1 s⁻¹, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messungen wurden jeweils nach 2 h und 24 h durchgeführt. Zwischen diesen Zeitpunkten wurde die Paste bei 25 °C gelagert.

In den beiden folgenden Tabellen sind für die Schergeschwindigkeiten von 1,06 s⁻¹ und 118 s⁻¹ jeweils die nach den angegebenen Lagerzeiten erhaltenen entsprechenden Viskositätswerte aufgeführt.

**Tabelle 2: Schergeschwindigkeit 1,06 s⁻¹ (Angaben der Viskositäten in Pa*s)**

| **Rezeptur** | **A** | **B** | **C** | **D** |
|---|---|---|---|---|
| **2 h** | 0,66 | 0,69 | 2,06 | 2,13 |
| **24 h** | 0,80 | 0,82 | 2,34 | 2,40 |

**Tabelle 3: Schergeschwindigkeit 118 s⁻¹ (Angaben der Viskositäten in Pa*s)**

| **Rezeptur** | **A** | **B** | **C** | **D** |
|---|---|---|---|---|
| **2 h** | 0,64 | 0,65 | 2,79 | 2,84 |
| **24 h** | 0,72 | 0,74 | 3,02 | 3,12 |

Die Viskositäten der zu vergleichenden Plastisole A und B bzw. C und D unterschieden sich praktisch nicht.

### Beispiel 6: Geliereigenschaften der nach Beispiel 4 hergestellten Plastisole

Die Untersuchung des Gelierverhaltens der Plastisole wurde in einem Oszillationsviskosimeter der Marke Bohlin CVO (Meßsystem PP20), welches schubspannungsgesteuert betrieben wurde, vorgenommen.
Folgende Parameter wurden eingestellt:
- Modus:: Temperatur-Gradient Start-Temperatur: 25°C End-Temperatur: 180°C Heiz/Kühlrate: 2°C/min Temperatur nach der Messung: 25°C Oszillations-Frequenz: 2 Hz Verzögerungszeit: 1 s Wartezeit: 15 s Kontinuierliche Oszillation: an Automatische Schubspannungsvorgabe: an Startschubspannung: 0,3 Pa Soll-Deformation: 0,002 Spaltweite 0,5 mm

### Durchführung des Messvorganges:

Auf die untere Messsystemplatte wurde mit dem Spatel ein Tropfen des zu messenden Plastisols (Rezepturen C und D aus Beispiel 4) luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Schutzabdeckung, die auch der Wärmeisolierung dient, aufgelegt und die Messung gestartet.

Aufgetragen wurde die "komplexe Viskosität" des Plastisols in Abhängigkeit von der Temperatur. Ein Einsetzen des Geliervorganges ist in einem plötzlichen starken Anstieg der komplexen Viskosität zu erkennen. Je früher dieser Viskositätsanstieg einsetzt, desto besser ist die Gelierfähigkeit des Systems.

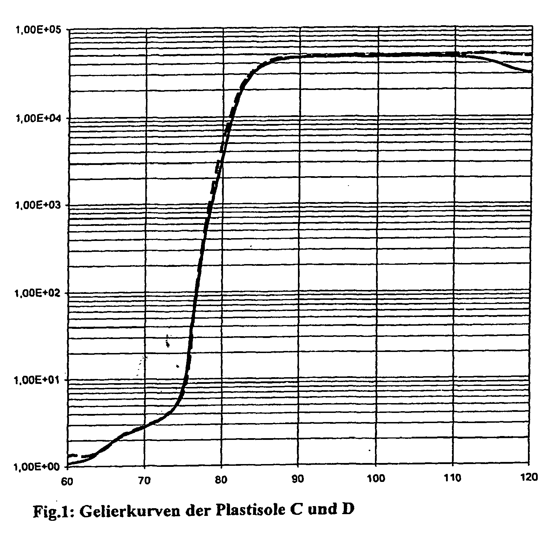

In Fig. 1 ist der für das Einsetzen der Gelierung relevante Ausschnitt des Viskositäts-Temperatur-Verlaufs ("Gelierkurve") aufgeführt. Die Y-Achse zeigt die komplexen Viskositäten in Pa·s, die X-Achse die Temperaturen in °C. Die durchgezogene Linie bezeichnet Plastisol C (mit erfindungsgemäßem Isodecyl-Benzoat), die unterbrochene Linie bezeichnet Plastisol D (mit methylverzweigtern Isodecylbenzoat, Vergleichsbeispiel).

Es ist zu erkennen, dass die beiden Kurven praktisch deckungsgleich verlaufen, so dass für beide Isodecylbenzoat-Typen von einem vergleichbaren Gelierverhalten ausgegangen werden kann

### Beispiel 7: Bestimmung der Glasübergangstemperatur der beiden Benzoate

Durch die Zugabe eines Weichmachers wird die Glasübergangstemperatur von PVC so weit emiedrigt, dass der Werkstoff auch noch bei tiefen Temperaturen flexibel ist. Die Fähigkeit eines Weichmachers, die Glasübergangstemperatur T_{G} abzusenken, ist umso größer, je niedriger die Glasübergangstemperatur eines als Weichmacher geeigneten Stoffes ist.

Eine Messung der Glasübergangstemperatur des reinen Weichmachers erlaubt somit Rückschlüsse auf dessen Kälteflexibilisierungspotenzial.

Zur Bestimmung von T_{G} können beispielsweise die Dynamische Differenz-Kalorimetrie (DDK) oder die Torsional Braid Analysis (TBA) verwendet werden. In den hier beschriebenen Fällen wurde auf Grund der höheren Genauigkeit die TBA-Methode durchgeführt. Diese ist eine Variante der z.B. in DIN EN ISO 6721 Teil 2 beschriebenen "klassischen" Torsionsschwingungsanalyse (TSA). Bei TBA wurde das zu untersuchende Material (hier die in Beispiel 2 und 3 genannten Produkte) auf einen in Litzenform geflochtenen und entschlichteten Glasfaser-Roving (Beladung zwischen 18 und 25 Gew. %) aufgebracht. An diesem wurde im Torsionspendel (MYRENNE ATM III) bei Temperaturen zwischen -180 und +100 °C und einer Frequenz von 1 s⁻¹ jeweils die Steifigkeit G' und der Verlustmodul G" bestimmt. Aus dem Maximum von G" ließ sich die Glasübergangstemperatur T_{G} bestimmen.
Für das nach Beispiel 2 erfindungsgemäß hergestellte Isodecylbenzoat wurde ein T_{G} von - 92 °C bstimmt. Für das Vergleichsbeispiel, den ausschließlich Methylgruppen verzweigten Isodecylester aus Beispiel 3 wurde ein T_{G} von -90 °C ermittelt.
Bei einer Methodengenauigkeit von 0,5 bis 1 °C ist dieser Unterschied daher als signifikant zu betrachten.

Zusammenfassend lässt sich sagen, dass die erfindungsgemäßen Isodecylbenzoate sich von dem Vergleichsbeispiel im Wesentlichen in einer verbesserten Kälteflexibilisierung, ausgedrückt durch den Glaspunkt T_{G} des Weichmachers, und einem durch die andere Rohstoffbasis für die Alkohole bedingten wirtschaftlicheren Herstellprozess unterscheiden.

## Patentansprüche

1. Gemisch isomerer Benzoesäuredecylester, enthaltend
50 - 99 % Benzoesäure-2-propylheptylester und
1 - 50% Benzoesäure-2-isopropyl-4-methyl-hexylester und/oder Benzoesäure-2-isopropyl-5-methyl-hexylester, Benzoesäure-2-propyl-4-methylhexylester, Benzoesäure-2-propyl-5-methylhexylester.

2. Gemisch enthaltend 5 bis 90 Gew.-% isomere Benzoesäure decylester gemäß Anspruch 1 und 10 bis 95 Gew.-% Phthalsäuredialkylester, wobei deren Alkylreste 4 bis 13 Kohlenstoffatome enthalten

3. Gemisch nach Anspruch 2,
**dadurch gekennzeichnet,**
**dass** als Phthalsäuredialkylester Phthalsäurediisononylester eingesetzt werden.

4. Gemisch enthaltend
5 bis 90 Gew.-% isomere Benzoesäuredecylester nach Anspruch 1 und
10 bis 95 Gew.-% Adipinsäuredialkylester, wobei deren Alkylreste 4 bis 13 Kohlenstoffatome enthalten.

5. Gemisch nach Anspruch 4,
**dadurch gekennzeichnet,**
**dass** als Adipinsäuredialkylester Adipinsäurediisononylester eingesetzt werden.

6. Gemische enthaltend 5 bis 90 Gew.-% isomere Benzoesäuredecylester gemäß Anspruch 1 und 10 bis 95 Gew.% Cyclohexandicarbonsäurealkylester, wobei deren Alkylreste 4 bis 13 Kohlenstoffatome enthalten.

7. Gemisch nach Anspruch 6,
**dadurch gekennzeichnet,**
**dass** als Cyclohexandicarbonsäurealkylester Cyclohexandicarbonsäurediisononylester eingesetzt werden.

8. Verfahren zur Herstellung eines Gemisches isomerer Benzoesäuredecylester, enthaltend 50 - 99 % Benzoesäure-2-propylheptylester und 1 - 50% Benzoesäure-2-isopropyl-4-methyl-hexylester und/oder Benzoesäure-2-isopropyl-5-methyl-hexylester, Benzoesäure-2-propyl-4-methylhexylester, Benzoesäure-2-propyl-5-methylhexylester,
**dadurch gekennzeichnet,**
**dass** 2-Propylheptanol, 2-Isopropyl-4-methyl-hexanol und/oder 2-Isopropyl-5-methyl-hexanol, 2-Propyl-4-methylhexanol, 2-Propyl-5-methylhexanol mit Benzoesäure verestert werden

9. Verfahren zur Herstellung eines Gemisches isomerer Benzoesäuredecylester, enthaltend 50 - 99 % Benzoesäure-2-propylheptylester und 1 - 50% Benzoesäure-2-isopropyl-4-methyl-hexylester und/oder Benzoesäure-2-isopropyl-5-methyl-hexylester, Benzoesäure-2-propyl-4-methylhexylester, Henzoesäure-2-propyl-5-methylhexylester,
**dadurch gekennzeichnet,**
**dass** Benzoesäuremethylester, Benzoesäureethylester, Benzoesäurepropanester, Benzoesäurebutanester mit 2-Propylheptanol, 2-Isopropyl-4-methyl-hexanol und/oder 2-Isopropyl-5-methyl-hexanol, 2-Propyl-4-methylhexanol, 2-Propyl-5-methylhexanol umgeestert werden.

10. Verwendung der Gemische nach einem der Ansprüche 1 bis 7 als Weichmacher für Polymere, PVC oder PVC-Plastisole.

11. Verwendung der Gemische nach einem der Ansprüche 1 bis 7 in Farben und Lacken.

12. Verwendung der Gemische nach einem der Ansprüche 1 bis 7 in Klebstoffen, Klebstoffkomponenten oder Dichtungsmassen.

## Claims

1. A mixture of isomeric decyl benzoates containing from 50 to 99% of 2-propylheptyl benzoate and from 1 to 50% of 2-isopropyl-4-methylhexyl benzoate and/or 2-isopropyl-5-methylhexyl benzoate, 2-propyl-4-methylhexyl benzoate, or 2-propyl-5-methylhexyl benzoate.

2. A mixture containing from 5 to 90% by weight of isomeric decyl benzoate according to claim 1 and from 10 to 95% by weight of dialkyl phthalates whose alkyl radicals contain from 4 to 13 carbon atoms.

3. A mixture according to claim 2,
**characterized in that**
diisononyl phthalates are used as the dialkyl phthalates.

4. A mixture containing
from 5 to 90% by weight of isomeric decyl benzoates according to claim 1 and from 10 to 95% by weight of dialkyl adipates whose alkyl radicals contain from 4 to 13 carbon atoms.

5. A mixture according to claim 4,
**characterized in that**
diisononyl adipates are used as the dialkyl adipates.

6. A mixture containing from 5 to 90% by weight of isomeric decyl benzoates according to claim 1 and from 10 to 95% by weight of alkyl cyclohexanedicarboxylates whose alkyl radicals contain from 4 to 13 carbon atoms.

7. A mixture according to claim 6,
**characterized in that**
diisononyl cyclohexanedicarboxylates are used as alkyl cyclohexanedicarboxylates.

8. A process for preparing a mixture of isomeric decyl benzoates, containing from 50 to 99% of 2-propylheptyl benzoate and from 1 to 50% of 2-isopropyl-4-methylhexyl benzoate and/or 2-isopropyl-5-methylhexyl benzoate, 2-propyl-4-methylhexyl benzoate or 2-propyl-5-methylhexyl benzoate,
**characterized in that**
benzoic acid is used to esterify 2-propylheptanol, 2-isopropyl-4-methylhexanol and/or 2-isopropyl-5-methylhexanol, 2-propyl-4-methylhexanolor2-propyl-5-methylhexanol.

9. A process for preparing a mixture of isomeric decyl benzoates, containing from 50 to 99% of 2-propylheptyl benzoate and from 1 to 50% of 2-isopropyl-4-methylhexyl benzoate and/or 2-isopropyl-5-methylhexyl benzoate, 2-propyl-4-methylhexyl benzoate or 2-propyl-5-methylhexyl benzoate,
**characterized in that**
2-propylheptanol, 2-isopropyl-4-methylhexanol and/or 2-isopropyl-5-methylhexanol, 2-propyl-4-methylhexanolor 2-propyl-5-methylhexanol are used to transesterify methyl benzoate, ethyl benzoate, propyl benzoate or butyl benzoate.

10. The use of the mixtures according to any one of claims 1 to 7 as plasticizers for polymers, PVC or PVC plastisols.

11. The use of the mixtures according to any one of claims 1 to 7 in paints, inks or coating materials.

12. The use of the mixtures according to any one of claims 1 to 7 in adhesives, in components of adhesives or in sealing compounds.

## Revendications

1. Mélange d'esters décyliques de l'acide benzoïque isomères, contenant
50 - 99 % d'ester 2-propylheptylique de l'acide benzoïque et
1 - 50 % d'ester 2-isopropyl-4-méthylhexylique de l'acide benzoïque et/ou d'ester 2-isopropyl-5-méthylhexylique de l'acide benzoïque, d'ester 2-propyl-4-méthylhexylique de l'acide benzoïque, d'ester 2-propyl-5-mëthylhexylique de l'acide benzoïque.

2. Mélange contenant 5 à 90 % en poids d'esters décyliques de l'acide benzoïque isomères selon la revendication 1, et 10 à 95 % en poids d'ester dialkylique de l'acide phtalique, dont les radicaux alkyle comportent 4 à 13 atomes de carbone.

3. Mélange selon la revendication 2,
**caractérisé en ce que**
comme ester dialkylique de l'acide phtalique on utilise l'ester diisononylique de l'acide phtalique.

4. Mélange contenant
5 - 90 % en poids d'esters décyliques de l'acide benzoïque isomères selon la revendication 1, et
10 à 95 % en poids d'ester dialkylique de l'acide adipique, dont les radicaux alkyle comportent 4 à 13 atomes de carbone.

5. Mélange selon la revendication 4,
**caractérisé en ce que**
comme ester dialkylique de l'acide adipique on utilise l'ester düsononylique de l'acide adipique.

6. Mélanges contenant 5 à 90 % en poids d'esters décyliques de l'acide benzoïque isomères selon la revendication 1, et 10 à 95 % en poids d'ester alkylique de l'acide cyclohexanedicarboxylique, dont les radicaux alkyle contiennent 4 à 13 atomes de carbone.

7. Mélange selon la revendication 6,
**caractérisé en ce que**
comme ester alkylique de l'acide cyclohexanedicarboxylique on utilise l'ester diisononylique de l'acide çyclohexanedicarboxylique.

8. Procédé pour la préparation d'un mélange d'esters décyliques de l'acide benzoïque isomères contenant 50 - 99 % d'ester 2-propylheptylique de l'acide benzoïque, et 1 - 50 % d'ester 2-isopropyl-4-méthylhexylique de l'acide benzoïque et/ou d'ester 2-isopropyl-5-méthylhexylique de l'acide benzoïque, d'ester 2-propyl-4-méthylhexylique de l'acide benzoïque, d'ester 2-propyl-5-méthylhexylique de l'acide benzoïque,
**caractérisé en ce que**
le 2-propylheptanol, le 2-isopropyl-4-méthyl-hexanol et/ou le 2-isopropyl-5-méthyl-hexanol, le 2-propyl-4-méthylhexanol, le 2-propyl-5-méthylhexanol sont estérifiés avec de l'acide benzoïque.

9. Procédé pour la préparation d'un mélange d'esters décyliques de l'acide benzoïque isomères contenant 50 - 99 % d'ester 2-propylheptylique de l'acide benzoïque, et 1 - 50 % d'ester 2-isopropyl-4-méthylhexylique de l'acide benzoïque et/ou d'ester 2-isopropyl-5-méthyl-hexylique de l'acide benzoïque, d'ester 2-propyl-4-méthylhexylique de l'acide benzoïque, d'ester 2-propyl-5-méthylhexylique de l'acide benzoïque,
**caractérisé en ce que**
l'ester méthylique de l'acide benzoïque, l'ester éthylique de l'acide benzoïque, l'ester propanoïque de l'acide benzoïque, l'ester butanoïque de l'acide benzoïque sont trans-estérifiés avec le 2-propylheptanol, le 2-isopropyl-4-méthylhexanol, et/ou le 2-isopropyl-5-méthylhexanol, le 2-propyl-4-méthylhexanol, le 2-propyl-5-méthylhexanol.

10. Utilisation des mélanges selon l'une des revendications 1 à 7. comme plastifiants pour polymères. PVC ou plastisols de PVC.

11. Utilisation des mélanges selon l'une des revendications 1 à 7. dans des peintures et vernis.

12. Utilisation des mélanges selon l'une des revendications 1 à 7, dans des adhésifs, des composants d'adhésifs ou des masses d'étanchéité.
